# EUROPEAN PATENT APPLICATION

(11) **EP 2 631 831 A1**
(43) Date of publication of application: **28.08.2013**
(21) Application number: 13157009.5
(22) Date of filing: 27.02.2013
(51) Int. Cl.: G06F 19/00, G06Q 50/22, G06Q 50/24

(54) **Method and Apparatus for Managing Patients Using Group Communication**

(30) Priority: 27.02.2012 KR 20120019820
(71) Applicant: Samsung Electronics Co., Ltd, Gyeonggi-do 443-742 (KR)
(72) Inventor: Lee, Joo-Yeol, 443-742 Gyeonggi-do (KR)
(74) Representative: Birchenough, Lewis

(57) **Abstract**

A group communication-based patient management method for patient management by a patient management server by requesting connection of a first call between a patient terminal and a first management terminal corresponding to a representative contact in a patient management group list, and a second call between the patient terminal and a second management terminal corresponding to a general contact in the patient management group list, connecting calls and transmitting connection state information associated with the call connection, to one of the management terminals, whose call to the patient terminal is not connected, checking call result information upon termination of the call connection between the patient terminal and the management terminal, and transmitting the call result information to the management terminals, whose call to the patient terminal is not connected.

## Description

### BACKGROUND OF THE INVENTION

### 1. Field of the Invention

The present invention relates generally to a technique for managing patients using communication technologies, and more particularly, to a technique for managing a patient's condition using a group call between a terminal (or patient terminal) used by a patient and terminals (or management terminals) used by managers.

### 2. Description of the Related Art

Generally, a call protocol, a connection between a transmitting terminal and a receiving terminal, may end on a one-time basis regardless of its type with establishment and termination of a call.

A patient (e.g., an autistic patient) who requires ongoing management may feel uneasy when physically separated from a caregiver (e.g., a parent or family member), so the patient may often need communication with a high-priority caregiver (e.g., mother) who provides comfort and managers of patients requiring ongoing management may be burdened by calls from a patient.

If a call connection to his or her high-priority caregiver is unavailable, the patient may become anxious or may be in danger. Therefore, there is a need for a method capable of informing the second or third manager of the call failure if the patient's call connection to the high-priority caregiver failed. In addition, there is a demand for a method capable of connecting with the second or third manager.

### SUMMARY OF THE INVENTION

Therefore, the present invention has been made to solve the above-stated problems occurring in the prior art, and the present invention provides a method and apparatus for grouping a patient terminal used by a patient and management terminals used by managers, and connecting the patient terminal to the management terminals, making it possible to easily share information about the connection state to the patient terminal and the patient's condition.

According to one aspect of the present invention, there is provided a group communication-based patient management method for operating patient management by a patient management. The method includes requesting connection of a first call between a patient terminal and a first management terminal corresponding to a representative contact in a patient management group list, and a second call between the patient terminal and a second management terminal corresponding to a general contact in the patient management group list, connecting a call between the patient terminal and at least one of the first management terminal and second management terminal, transmitting connection state information associated with the call connection, to one of the first management terminal and second management terminal, whose call to the patient terminal is not connected, if termination of the call connection between the patient terminal and the at least one of the first management terminal and second management terminal, checking call result information, and transmitting the call result information to the one of the first management terminal and second management terminal, whose call to the patient terminal is not connected.

According to another aspect of the present invention, there is provided a group communication-based patient management method for operating patient management by a management terminal using a patient terminal used by a patient, and the management terminal used by a user, which manages a patient and is included in a patient management group. The method includes, upon receiving a request for connection of a second call to the patient terminal, performing connection of a second call to the patient terminal, receiving connection state information associated with connection of a first call between the patient terminal and a first management terminal, providing the connection state information to the user, and receiving call result information and providing the received call result information to the user.

According to further another aspect of the present invention, there is provided a group communication-based patient management server apparatus for managing a patient, using a patient terminal used by a patient, a management terminal used by a user, which manages a patient and is included in a patient management group, and a patient management server situated between the patient terminal and the management terminal, for controlling a connection between the patient terminal and the management terminal. The apparatus includes a call connection manager for requesting connection of a first call between a patient terminal and a first management terminal corresponding to a representative contact in a patient management group list, and a second call between the patient terminal and a second management terminal corresponding to a general contact in the patient management group list, and connecting a call between the patient terminal and at least one of the first management terminal and second management terminal, a call connection information manager for checking connection state information associated with the call connection, and transmitting the connection state information associated with the call connection to one of the first management terminal and second management terminal, whose call to the patient terminal is not connected, and a call result information manager for, upon termination of the call connection between the patient terminal and the at least one of the first management terminal and second management terminal, checking call result information and providing the call result information to one of the first management terminal and second management terminal, whose call to the patient terminal is not connected.

According to yet another aspect of the present invention, there is provided a group communication-based patient management terminal apparatus for managing a patient, using a patient terminal used by a patient, a management terminal used by a user, which manages a patient and is included in a patient management group, and a patient management server situated between the patient terminal and the management terminal, for controlling a connection between the patient terminal and the management terminal. The apparatus includes a call manager for, upon receiving a message for requesting call connection from the patient management server, checking information included in the message for requesting call connection to determine whether the requested call is a group call to management terminals corresponding to the patient management group, whether the request call is a first call, whether the request call is a second call, and whether to start a session for connection of the second call, and requesting connection of the first or second call according thereto, a call connector for, upon request of the call manager, connecting the first or second call to the patient terminal and starting a session for connection of the second call, and a call result information manager for, upon termination of the call connection to the patient terminal, receiving call result information from the user and providing the received call result information to the patient management server.

### BRIEF DESCRIPTION OF THE DRAWINGS

The above and other aspects, features and advantages of the present invention will be more apparent from the following description taken in conjunction with the accompanying drawings, in which:
FIG. 1 is a diagram illustrating a configuration of a patient management system, to which a group communication-based patient management method is applied, according to an embodiment of the present invention;
FIG. 2 is a block diagram illustrating a structure of a patient terminal, to which a group communication-based patient management method is applied, according to an embodiment of the present invention;
FIG. 3 is a block diagram illustrating a structure of a patient management server, to which a group communication-based patient management method is applied, according to an embodiment of the present invention;
FIG. 4 is a block diagram illustrating a structure of a management terminal, to which a group communication-based patient management method is applied, according to an embodiment of the present invention;
FIG. 5 is a signal flow diagram illustrating a group communication-based patient management method according to an embodiment of the present invention;
FIG. 6A is a signal flow diagram illustrating an embodiment of the process of requesting connection of a group call in FIG. 5;
FIG. 6B is a signal flow diagram illustrating another embodiment of the process of requesting connection of a group call in FIG. 5;
FIG. 6C is a signal flow diagram illustrating a further embodiment of the process of requesting connection of a group call in FIG. 5;
FIG. 7A is a signal flow diagram illustrating an embodiment of the process of connecting a call in FIG. 5;
FIG. 7B is a signal flow diagram illustrating another embodiment of the process of connecting a call in FIG. 5; and
FIG. 7C is a signal flow diagram illustrating a further embodiment of the process of connecting a call in FIG. 5.

### DETAILED DESCRIPTION OF EMBODIMENTS OF THE PRESENT INVENTION

Various embodiments of the present invention will be described in detail with reference to the accompanying drawings. In the following description, specific details such as detailed configurations and components are merely provided to assist the overall understanding of the embodiments of the present invention. Therefore, it should be apparent to those skilled in the art that various changes and modifications of the embodiments described herein can be made without departing from the scope and spirit of the invention. The same drawing reference numerals will be used to refer to the same elements, features and structures throughout the drawings.

In addition, detailed descriptions of well-known functions and constructions are omitted to avoid obscuring the description of the present invention.

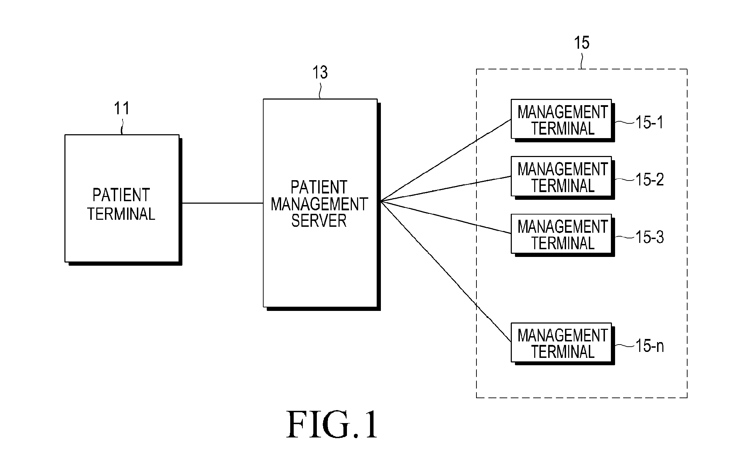

FIG. 1 is a diagram illustrating a configuration of a patient management system, to which a group communication-based patient management method is applied according to an embodiment of the present invention.

Referring to FIG. 1, the patient management system, according to an embodiment of the present invention, includes a patient terminal 11 used by a patient, a patient management server 13 that provides a group communication-based patient management service, and a plurality of management terminals 15 used by users (or managers), which manage a patient and are included (or registered) in a patient management group.

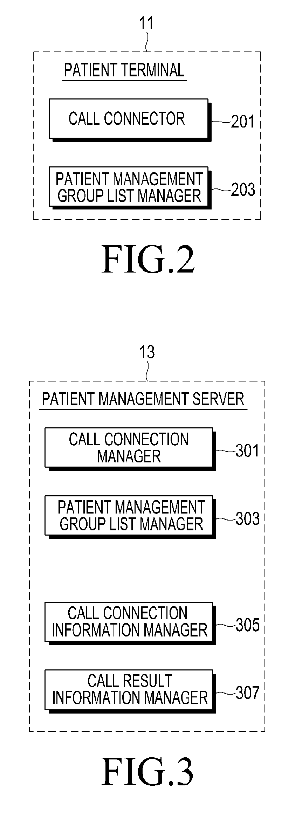

The patient terminal 11, which performs a call function, stores and manages a list of the plurality of management terminals 15 corresponding to the patient management group. Specifically, as illustrated in FIG. 2, the patient terminal 11 includes a call connector 201 and a patient management group list manager 203. The call connector 201 handles a call function, and the patient management group list manager 203 stores and manages a list of the plurality of management terminals 15 corresponding to the patient management group.

The patient management group list manager 203 stores a plurality of contacts, selectively sets at least one of the stored contacts as a patient management group, manages the set patient management group, and sets at least one of a plurality of contacts included in the patient management group as a representative contact. The plurality of stored contacts included in the patient management group may be assigned priority to be used for attempting a call connection.

A list of contacts, which is set as a patient management group, may be defined as a "patient management group list." A contact included in the patient management group list and set as a representative contact may be defined as a "representative contact." A contact except for the representative contact among the contacts included in the patient management group list may be defined as a "general contact."

The call connector 201 is configured to connect a call to the plurality of management terminals 15 included in the patient management group list. If a user (or patient) enters a predetermined key for a call to a patient management group, the patient terminal 11 starts an operation of making a call to the patient management group. For example, the patient terminal 11 attempts connection of a first call to a first management terminal (e.g., 15-1) corresponding to the representative contact, and at the same time, attempts connection of a second call to a second management terminal (e.g., 15-2, 15-3, ..., 15-n) corresponding to the general contacts.

The first call, a call connection typically performed between terminals, may be an operation in which if the patient terminal 11 transmits a calling signal for requesting a call to the first management terminal (e.g., 15-1) and the calling signal arrives at the first management terminal (e.g., 15-1), then the first management terminal (e.g., 15-1) activates an alarm (e.g., ringtone, indication light, vibration, etc.) indicating the receipt of a call and a ringing signal by the first management terminal (e.g., 15-1) is transmitted to the patient terminal 11.

The second call is a call set to perform only an operation in which the patient terminal 11 transmits a calling signal for requesting a call to the second management terminals (e.g., 15-2, 15-3, ..., 15-n), and set not to perform an operation in which a ringing signal by the first management terminal (e.g., 15-1) is transmitted to the patient terminal 11.

Although the patient terminal 11 may manage the patient management group list, the patient management group list may also be stored and managed in the patient management server 13. Because the patient management group list is stored and managed in the patient management server 13, the operation, in which the patient terminal 11 makes a call to the patient management group when the user (or patient) enters a predetermined key for a call to the patient management group, may be modified in various ways. For example, if the user (or patient) enters a predetermined key for a call to the patient management group, the patient terminal 11 requests a patient management group list from the patient management server 13, receives the requested patient management group list from the patient management server 13, and makes connection of a first call to the first management terminal (e.g., 15-1) corresponding to the received patient management group list and a second call to the second management terminals (e.g., 15-2, 15-3, ..., 15-n) corresponding thereto. Alternatively, if the user (or patient) enters a predetermined key for a call to the patient management group, the patient terminal 11 requests a call connection to the patient management group from the patient management server 13, and the patient management server 13 checks contacts included in the patient management group stored therein and makes connection of a first call to the first management terminal (e.g., 15-1) corresponding to the contact included in the patient management group and a second call to the second management terminals (e.g., 15-2, 15-3, ..., 15-n) corresponding to the contacts included in the patient management group.

The patient management server 13, situated between the patient terminal 11 and the first management terminal (e.g., 15-1) and the second management terminals (e.g., 15-2, 15-3, ..., 15-n), manages the first call between the patient terminal 11 and the first management terminal (e.g., 15-1) and the second call between the patient terminal 11 and the second management terminals (e.g., 15-2, 15-3, ..., 15-n).

As illustrated in FIG. 3, the patient management server 13 includes a call connection manager 301, a call connection information manager 305, and a call result information manager 307. Optionally, the patient management server 13 may include a patient management group list manager 303 capable of storing and managing a patient management group list.

Specifically, upon receiving a request for a first call from the patient terminal 11, the call connection manager 301 transmits a calling signal to the first management terminal (e.g., 15-1) corresponding to a representative contact in the patient management group list, and as the calling signal arrives at the first management terminal (e.g., 15-1), the call connection manager 301 transmits a ringing signal received from the first management terminal (e.g., 15-1), to the patient terminal 11, thereby making connection of the first call. The call connection manager 301 measures elapsed time, starting from the time the calling signal is transmitted to the first management terminal (e.g., 15-1).

If the first management terminal (e.g., 15-1) sends a response to the call request, the call connection manager 301 establishes a call session between the patient terminal 11 and the first management terminal (e.g., 15-1). The call connection manager 301 generates and stores response time information including information about the time elapsed from the point the calling signal was transmitted, to the point the call is responded or answered.

If the elapsed time exceeds a predetermined first time, the call connection manager 301 transmits first state information including the elapsed time to the second management terminals (e.g., 15-2, 15-3, ..., 15-n). If there is no response to the call from the first management terminal (e.g., 15-1) even though the elapsed time has arrived at a predetermined second time, the call connection manager 301 transmits second state information to the second management terminals (e.g., 15-2, 15-3, ..., 15-n). The second state information may include information indicating the failure in the call connection to the first management terminal (e.g., 15-1).

The call connection manager 301 performs call connection to the second management terminals (e.g., 15-2, 15-3, ..., 15-n), as the call to the first management terminal (e.g., 15-1) is not connected. For example, the call connection manager 301 includes information for requesting call connection to the second management terminals (e.g., 15-2, 15-3, ..., 15-n) in the second state information, transmit the renewed second state information to the second management terminals (e.g., 15-2, 15-3, ..., 15-n), and establishes a call session to one of the plurality of second management terminals (e.g., 15-2, 15-3, ..., 15-n), which has first sent a response signal (e.g., Acknowledgement (ACK) signal), based on the chronological order in which the call connection manager 301 has received response signals (e.g., ACK signals) from the second management terminals (e.g., 15-2, 15-3, ..., 15-n). In this state, the patient terminal 11 is continuously transmitting a calling signal to the second management terminals (e.g., 15-2, 15-3, ..., 15-n). Accordingly, the call connection manager 301 sends a command to enable a call session to the second management terminal that will establish a call session, making it possible to connect a call session between the patient terminal 11 and the second management terminal. For example, the call connection manager 301 may send a command to enable a call session between the patient terminal 11 and the second management terminal, which have been connected by a calling signal, to the second management terminal that will establish the call session, and the second management terminal that will establish the call session may enable the call session, thereby making it possible to establish a call session between the patient terminal 11 and the second management terminal that will establish the call session.

Alternatively, the call connection manager 301 may send a command to enable a call session sequentially to the plurality of second management terminals (e.g., 15-2, 15-3, ..., 15-n) according to a predetermined priority rule, regardless of the response signal (e.g., ACK signal) from the second management terminals (e.g., 15-2, 15-3, ..., 15-n), making it possible to connect a call session between the patient terminal 11 and the second management terminal. The call connection manager 301 sends a command to enable a call session sequentially to the second management terminals (e.g., 15-2, 15-3, ..., 15-n) according to the priorities, which are stored therein along with their associated contacts in the patient management group list. Specifically, assuming that the higher priority is given in sequence to the second management terminal #1 15-2, the second management terminal #2 15-3, ..., the second management terminal #(n-1) 15-n, the call connection manager 301 first sends a command to enable a call session to the second management terminal #1 15-2 with the highest priority, and waits for a predetermined time (e.g., 10 seconds). Upon receiving a response signal ACK from the second management terminal #1 15-2 within the predetermined time (e.g., 10 seconds), the call connection manager 301 connects a call session between the patient terminal 11 and the second management terminal #1 15-2. However, upon failure to receive a response signal ACK from the second management terminal #1 15-2 within the predetermined time (e.g., 10 seconds), the call connection manager 301 sends a command to enable a call session to the second management terminal #2 15-3 with the next highest priority.

The call connection information manager 305 generates and manages connection state information associated with the call connections, and the call result information manager 307 generates and manages call result information associated with the call results. The connection state information may be information that the patient management server 13 checks in a call connection process, and may include information such as an identifier of a connected management terminal or an unconnectable management terminal, and an elapsed time for establishing connection. The call result information is information that the patient management server 13 receives from the management terminal 15 after the call to the management terminal 15 is terminated, and includes information about the patient's condition and information about a keyword of the conversation, and also includes the recorded audio (or video) information of the call or conversation.

The management terminal 15 serves as the first management terminal 15-1 or serves as the second management terminals 15-2, 15-3, ..., 15-n. The first management terminal 15-1 operates to transmit a ringing signal upon receipt of a calling signal, whereas the second management terminals 15-2, 15-3, ..., 15-n operate not to transmit a separate response signal even though they receive a calling signal. These first management terminal 15-1 and second management terminals 15-2, 15-3, ..., 15-n may be set to correspond to contacts included in the patient management group. While a management terminal corresponding to the representative contact in the patient management group may serve as the first management terminal 15-1, management terminals corresponding to the remaining contacts included in the patient management group except for the representative contact may serve as the second management terminals 15-2, 15-3, ..., 15-n.

Preferably, therefore, the management terminal 15 is configured to serve as both the first management terminal 15-1 and the second management terminals 15-2, 15-3, ..., 15-n.

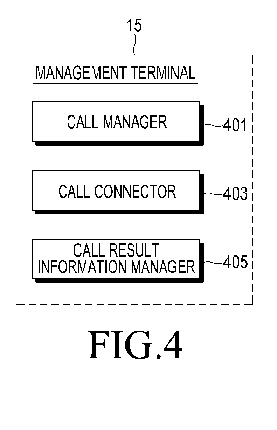

A structure of the management terminal 15 is described below with reference to FIG. 4 illustrating a structure of a management terminal, to which a group communication-based patient management method is applied according to an embodiment of the present invention.

The management terminal 15 includes a call manager 401 and a call connector 403.

A calling signal received from the outside is received via the call manager 401. The calling signal includes a management group call indicator indicating the group call to management terminals corresponding to the patient management group. The calling signal may selectively include one of first and second call indicators.

Accordingly, the call manager 401 checks the management group call indicator in the calling signal, and informs the call connector 403 of the group call to the management terminals corresponding to the patient management group. The call manager 401 checks if the calling signal includes a first call indicator or a second call indicator. If the calling signal includes a first call indicator, the call manager 401 instructs the call connector 403 to serve as the first management terminal 15-1. If the calling signal includes a second call indicator, the call manager 401 instructs the call connector 403 to serve as the second management terminals 15-2, 15-3, ..., 15-n.

Upon receiving from the call manager 401 an instruction to serve as the first management terminal 15-1, the call connector 403 checks an identifier of a terminal that transmitted the calling signal, provides an alarm ringtone, an indication light, vibration, etc. to the user of the management terminal 15 together with a contact corresponding to the identifier of the terminal, and replies to the first call to the patient terminal 11, i.e., transmits a ringing signal back to the patient terminal 11 that transmitted the calling signal.

Upon receiving from the call manager 401 an instruction to serve as the second management terminals 15-2, 15-3, ..., 15-n, the call connector 403 checks an identifier of a terminal that transmitted the calling signal, provides an alarm to the user of the management terminal 15 together with a contact corresponding to the identifier of the terminal, and maintains the connection by the calling signal, without replying to the second call to the patient terminal 11, i.e., without transmitting a ringing signal back to the patient terminal 11.

The call manager 401 receives second state information from the patient management server 13. Upon receiving the second state information, the call manager 401 sends a response signal ACK to the patient management server 13. The call manager 401 receives a request for connection of a call session to the patient terminal 11 from the patient management server 13. In response to the request, the call manager 401 requests the call connector 403 to start a call session to the patient terminal 11 being connected by the second call.

The second state information further includes an indicator for requesting connection of a call session to the patient terminal 11. Then, the call manager 401 requests the call connector 403 to start a call session to the patient terminal 11 being connected by the second call.

The management terminal 15 further includes a call result information manager 405. The call result information manager 405 is configured to perform different operations when the management terminal 15 serves as the first management terminal 15-1 and when it serves as the second management terminals 15-2, 15-3, ..., 15-n. When the management terminal 15 serves as the first management terminal 15-1, the call result information manager 405 requests the user to enter call result information upon request of the patient management server 13 or by himself or herself, if the call to the patient terminal 11 has ended. For example, by executing a User Interface (UI) on which information about the patient's condition and information about a keyword of the conversation may be entered, the call result information manager 405 receives call result information from the user and transmit the received call result information to the patient management server 13. However, when the management terminal 15 serves as the second management terminals 15-2, 15-3, ..., 15-n, the call result information manager 405 receives call result information from the patient management server 13, and display the received call result information on its screen together with an alarm that the user may recognize.

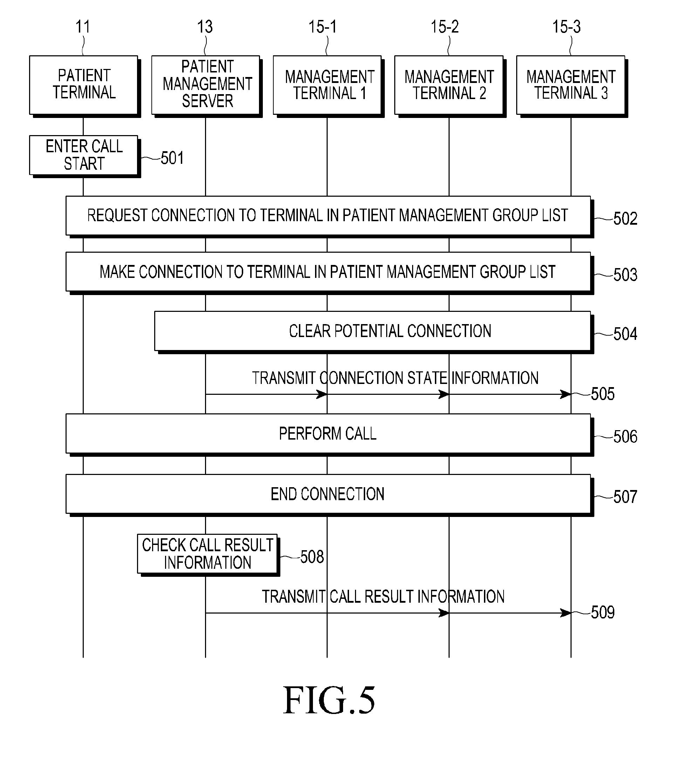

FIG. 5 is a signal flow diagram illustrating a group communication-based patient management method according to an embodiment of the present invention.

Referring to FIG. 5, in the group communication-based patient management method of the present invention, a call start is entered by a user of the patient terminal 11 in Step 501. For example, a predetermined key button or menu is input by the user of the patient terminal 11, for a group call to management terminals 15 (15-1, 15-2, and 15-3) corresponding to a patient management group. For the group call to the management terminals 15 corresponding to the patient management group, contacts included in the patient management group are stored and managed in the patient terminal 11. Here, the patient terminal 11 may provide, on a UI, its user with a menu on which contacts included in the patient management group is set. The user of the patient terminal 11 may set in advance the contacts included in the patient management group on the menu included in the patient terminal 11. Alternatively, contacts included in the patient management group may be stored and managed in the patient management server 13. Here, the patient management server 13 provides the user with the environment where he or she may manage contacts included in the patient management group on, for example, web pages, so the user may manage the contacts included in the patient management group on the web pages.

In Step 502, the patient terminal 11 requests a group call to the management terminals 15 corresponding to the patient management group, from the patient management server 13, and the patient management server 13 performs a process of requesting a call to the management terminals 15 corresponding to the patient management group.

The patient terminal 11 requests a first call to a first management terminal 15-1 and requesting a second call to at least one of second management terminals 15-2 and 15-3. Specifically, the patient management server 13 checks contacts included in the patient management group managed in the patient terminal 11 or the patient management server 13. The patient management server 13 transmits a calling signal for requesting the first call to the first management terminal 15-1 corresponding to the representative contact among the contacts included in the patient management group, to the first management terminal 15-1. Then, the first management terminal 15-1 checks or detects an identifier of the patient terminal 11 that transmitted the calling signal, a management group call indicator indicating the group call, and a first call indicator, from the calling signal for requesting the first call, provides an alarm (ringtone, indication light, vibration, etc.) to the user of the first management terminal 15-1 together with a contact corresponding to the identifier of the patient terminal 11 and an indication (e.g., text or icon indicating the group call to the patient terminal 11) indicating the group call, and replies to the first call to the patient terminal 11, i.e., transmits a ringing signal back to the patient terminal 11 that transmitted the calling signal. The patient management server 13 transmits a calling signal for requesting the second call to the second management terminals 15-2 and 15-3 corresponding to the remaining contacts except for the representative contact among the contacts included in the patient management group, to at least one of the second management terminals 15-2 and 15-3. Upon receiving the calling signal for requesting the second call, the second management terminals 15-2 and 15-3 check or detect an identifier of the patient terminal 11 that transmitted the calling signal, a management group call indicator indicating the group call, and a second call indicator, from the calling signal for requesting the second call. The second management terminals 15-2 and 15-3 provide an alarm (ringtone, indication light, vibration, etc.) to the users of the second management terminals 15-2 and 15-3 together with a contact corresponding to the identifier of the patient terminal 11 and an indication (e.g., text or icon indicating the group call to the patient terminal 11) indicating the group call, and maintain the connection by the calling signal, without replying to the second call to the patient terminal 11, i.e., without transmitting a ringing signal back to the patient terminal 11.

In Step 503, the patient terminal 11, the patient management server 13, the first management terminal 15-1, and the second management terminals 15-2 and 15-3 perform call connection. The patient management server 13 measures elapsed time, starting from the time the request for connection of the first call is performed in Step 502, and periodically transmits every predetermined first time (e.g., 10 seconds) the first state information indicating the absence of a response for the predetermined first time, to the second management terminals 15-2 and 15-3, if there is no response from the first management terminal 15-1. The patient management server 13 attempts connection to the second management terminals 15-2 and 15-3, if there is no response from the first management terminal 15-1 for a predetermined second time (e.g., 30 seconds). Connection to the second management terminals 15-2 and 15-3 may be achieved by requesting the second management terminals 15-2 and 15-3, which are potentially connected by the calling signal, to enable their connection session (or call session).

After a call to any one of the first management terminal 15-1 and the second management terminals 15-2 and 15-3 is connected, the patient management server 13 terminates the connection of the second management terminals 15-2 and 15-3 which are potentially connected, in Step 504.

In Step 505, the patient management server 13 checks connection state information as information about the call connection between patient terminal 11 and the management terminal 15, and transmits it to the unconnected management terminal 15 among the management terminals 15. The connection state information may be information that the patient management server 13 checks in a call connection process, and may include information such as an identifier of the connected management terminal 15 or unconnectable management terminal 15 and an elapsed time for establishing connection.

Thereafter, the patient management server 13 proceeds with a call between the patient terminal 11 and the management terminal 15 in Step 506, and ends the call in response to a call end request from the patient terminal 11 or the management terminal 15 in Step 507.

If the call between the patient terminal 11 and the management terminal 15 is ended, the patient management server 13 checks call result information in Step 508. Specifically, the patient management server 13 requests call result information from the management terminal 15 whose call is connected, and the management terminal 15 whose call is connected, requests its user to enter call result information including information about the patient's condition and information about a keyword of the conversation, and receives the requested call result information. The management terminal 15 whose call is connected, provides the received call result information to the patient management server 13.

In Step 509, the patient management server 13 provides the call result information to at least one management terminal 15 whose call is not connected.

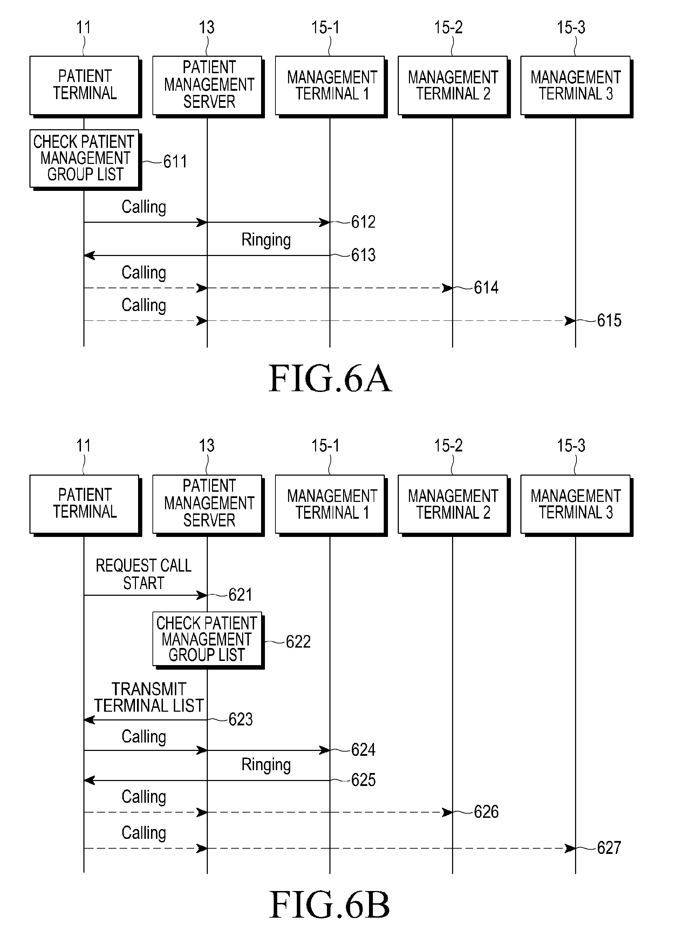

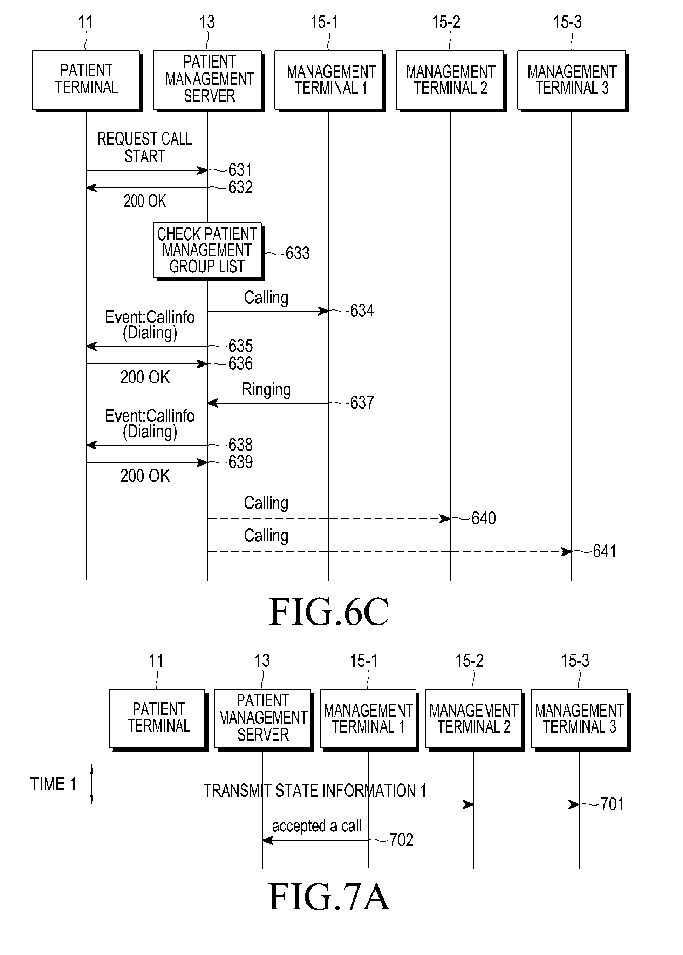

FIGs. 6A to 6C are signal flow diagrams illustrating a detailed process of Step 502 (i.e., a process of requesting a group call) in FIG. 5. FIG. 6A illustrates a first embodiment of Step 502 of requesting connection of a group call, FIG. 6B illustrates a second embodiment of Step 502 of requesting connection of a group call, and FIG. 6C illustrates a third embodiment of Step 502 of requesting connection of a group call.

Referring to FIG. 6A, the first embodiment of Step 502 of requesting connection of a group call illustrates a case where a list of a plurality of management terminals 15 corresponding to the patient management group is stored and managed in the patient terminal 11. According, in the first embodiment of Step 502 of requesting connection of a group call, after receiving a call start, the patient terminal 11 first checks a list of the plurality of management terminals 15 corresponding to the patient management group, which is stored in the patient terminal 11, in Step 611. The patient terminal 11 distinguishes a representative contact and a general contact in the list of the plurality of management terminals 15 corresponding to the patient management group.

In Step 162, the patient terminal 11 transmits a calling signal to the patient management server 13 to request connection of a first call to the first management terminal 15-1 corresponding to the representative contact, and the patient management server 13 forwards the calling signal to the first management terminal 15-1.

In Step 613, the first management terminal 15-1 checks or detects an identifier of the patient terminal 11 that transmitted the calling signal, a management group call indicator indicating the group call, and a first call indicator, from the calling signal for requesting the first call, provides an alarm (e.g., ringtone, indication light, vibration, etc.) to the user of the first management terminal 15-1 together with a contact corresponding to the identifier of the patient terminal 11 and an indication (e.g., text or icon indicating the group call to the patient terminal 11) indicating the group call, and replies to the first call to the patient terminal 11, i.e., transmits a ringing signal back to the patient terminal 11 that transmitted the calling signal.

In Steps 614 and 615, the patient terminal 11 transmits a calling signal for requesting the second call to the second management terminal 15-2 corresponding to a general contact in the list of the plurality of management terminals 15 corresponding to the patient management group, to at least one second management terminal 15-2, and upon receiving the calling signal for requesting the second call, the second management terminal 15-2 checks or detects an identifier of the patient terminal 11 that transmitted the calling signal, a management group call indicator indicating the group call, and a second call indicator, from the calling signal for requesting the second call. The second management terminal 15-2 provides an alarm (e.g., ringtone, indication light, vibration, etc.) to the user of the second management terminal 15-2 together with a contact corresponding to the identifier of the patient terminal 11 and an indication (e.g., text or icon indicating the group call to the patient terminal 11) indicating the group call, and maintains the connection by the calling signal, without replying to the second call to the patient terminal 11, i.e., without transmitting a ringing signal back to the patient terminal 11.

Referring to FIG. 6B, the second embodiment of Step 502 of requesting connection of a group call illustrates a case where a list of a plurality of management terminals 15 corresponding to the patient management group is managed by the patient management server 13.

In Step 621, after receiving a call start, the patient terminal 11 first requests a call start from the patient management server 13. In Step 622, the patient management server 13 checks a list of a plurality of management terminals 15 corresponding to the patient management group, stored therein. In Step 623, the patient management server 13 transmits the checked list of the plurality of management terminals 15 corresponding to the patient management group, back to the patient terminal 11.

Next, the patient terminal 11 performs Steps 624, 625, 626 and 627 of performing the first and second calls to the first management terminal 15-1 and the second management terminals 15-2 and 15-3, taking into account the list of the plurality of management terminals 15 corresponding to the patient management group. A detailed operation of Steps 624, 625, 626 and 627 may be performed in the same way as the operation in the first embodiment of Step 502 of requesting connection of a group call, illustrated in FIG. 6A.

Referring to FIG. 6C, the third embodiment of Step 502 of requesting connection of a group call illustrates a case where a list of a plurality of management terminals 15 corresponding to the patient management group is managed by the patient management server 13. However, in the third embodiment of Step 502 of requesting connection of a group call, a call request is initiated by the patient management server 13, unlike described in FIG. 6B.

Specifically, in Step 631, after receiving a call start, the patient terminal 11 first requests a call start from the patient management server 13. In response, the patient management server 13 sends a response signal "200 OK" to the patient terminal 13 in Step 632, indicating a successful request. In Step 633, the patient management server 13 checks a list of a plurality of management terminals 15 corresponding to the patient management group, stored therein. Next, the patient management server 13 transmits a calling signal for requesting the first call to the first management terminal 15-1 in Step 634, and at the same time, sends an event message including Callinfo for Dialing to the patient terminal 11 in Step 635. In response thereto, the patient terminal 11 sends a response signal "200 OK" to the patient management server 13 in Step 636. In Step 637, the first management terminal 15-1 checks or detects an identifier of the patient terminal 11 that transmitted the calling signal, a management group call indicator indicating the group call, and a first call indicator, from the calling signal for requesting the first call, provides an alarm (e.g., ringtone, indication light, vibration, etc.) to the user of the first management terminal 15-1 together with a contact corresponding to the identifier of the patient terminal 11 and an indication (e.g., text or icon indicating the group call to the patient terminal 11) indicating the group call, and replies to the first call to the patient terminal 11, i.e., transmits a ringing signal back to the patient terminal 11 that transmitted the calling signal. Next, the patient management server 13 sends an event message including Callinfo for Ringing to the patient terminal 11 in Step 638, and the patient terminal 11 sends a response signal "200 OK" to the patient management server 13 in Step 639. The patient management server 13 transmits a calling signal for requesting a second call to the second management terminals 15-2 and 15-3 corresponding to general contacts to at least one of the second management terminals 15-2 and 15-3, and upon receiving the calling signal for requesting the second call, the second management terminals 15-2 and 15-3 check or detect an identifier of the patient terminal 11 that transmitted the calling signal, a management group call indicator indicating the group call, and a second call indicator, from the calling signal for requesting the second call. In Steps 640 and 641, the patient management server 13 provides an alarm (e.g., ringtone, indication light, vibration, etc.) to the users of the second management terminals 15-2 and 15-3 together with a contact corresponding to the identifier of the patient terminal 11 and an indication (e.g., text or icon indicating the group call to the patient terminal 11) indicating the group call, and maintains the connection by the calling signal, without replying to the second call to the patient terminal 11, i.e., without transmitting a ringing signal back to the patient terminal 11.

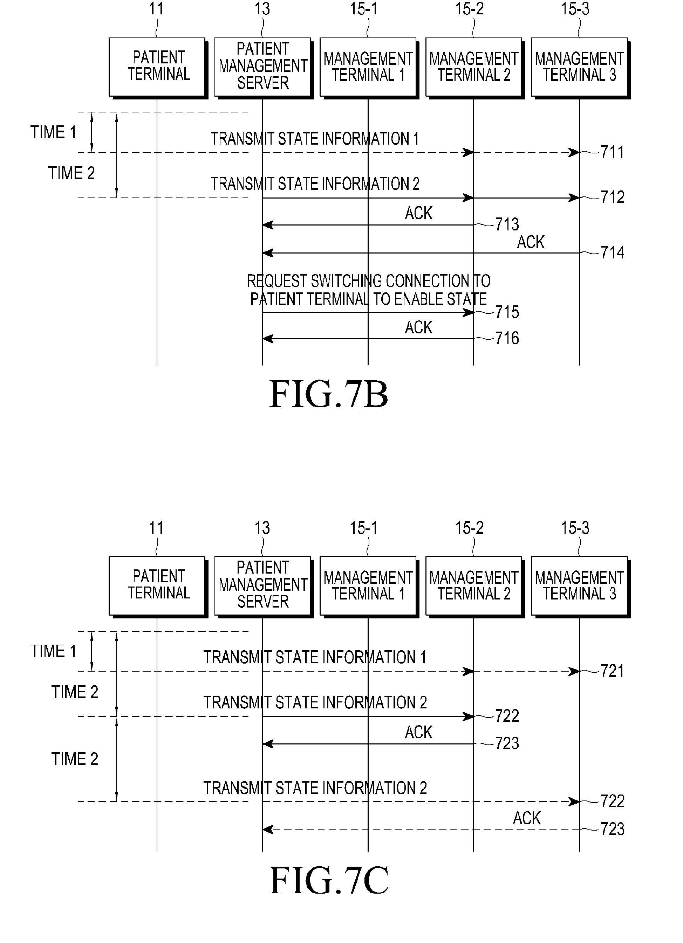

FIGs. 7A to 7C are signal flow diagrams illustrating a detailed process of Step 503 (i.e., a process of connecting a call) in FIG. 5. FIG. 7A illustrates a first embodiment of Step 503 of connecting a call, FIG. 7B illustrates a second embodiment of Step 503 of connecting a call, and FIG. 7C illustrates a third embodiment of Step 503 of connecting a call.

Referring to FIG. 7A, the first embodiment of Step 503 of connecting a call illustrates a case where the first management terminal 15-1 receives a call response from the user after a lapse of a predetermined first time.

The patient management server 13 continuously measures elapsed time, starting from the time it transmitted the calling signal to the first management terminal 15-1, and transmits first state information to the second management terminals 15-2 and 15-3 in Step 701, if the predetermined first time (e.g., 10 seconds) has elapsed, without receiving a call response signal from the user. In Step 702, upon receiving a call response signal from the user before arrival at a predetermined second time, the first management terminal 15-1 transmits a call response signal to the patient management server 13.

Referring to FIG. 7B, the second embodiment of Step 503 of connecting a call illustrates a case where the first management terminal 15-1 has not received a call response from the user even after a lapse of a predetermined second time.

As in the first embodiment, the patient management server 13 continuously measures elapsed time, starting from the time it transmitted the calling signal to the first management terminal 15-1, and transmits first state information to the second management terminals 15-2 and 15-3 in Step 711, if the predetermined first time (e.g., 10 seconds) has elapsed, without receiving a call response signal from the user. Upon arrival at a predetermined second time, the patient management server 13 attempts a call to the second management terminals 15-2 and 15-3, determining that the first management terminal 15-1 cannot respond to the call. Specifically, in Step 712, the patient management server 13 transmits second state information to the second management terminals 15-2 and 15-3 simultaneously. The second state information may include information indicating the failure in connection to the first management terminal 15-1, and information for requesting connection to the patient terminal 11. In response, upon receiving the second state information, the second management terminals 15-2 and 15-3 transmit a response signal ACK to the patient management server 13 in Steps 713 and 714. In Step 715, the patient management server 13 determines the second management terminal 15-2 corresponding to the first received response signal among the response signals ACKs received from the second management terminals 15-2 and 15-3, and sends a message for requesting to start the second call to the patient terminal 11, to the determined second management terminal 15-2. In Step 716, upon receiving the message, the second management terminal 15-2 enables call connection to the patient terminal 11 that has been connected through the second call, and transmits a response signal ACK to the patient management server 13.

Although not shown, if the first management terminal 15-1 is not connected even after a lapse of the first or second time, the patient management server 13 may attempt connection to the second management terminals 15-2 and 15-3 and notify the patient terminal 11 of its ongoing attempt to connect with the second management terminals 15-2 and 15-3. Here, the patient management server 13 transmits a ringing signal to the patient terminal 11.

After receiving the enable request of Step 715, the second management terminal 15-2 may provide an alarm (e.g., ringtone, indication light, vibration, etc.) for informing the user of the second management terminal 15-2 of its receipt of the enable request.

Referring to FIG. 7C, the third embodiment of Step 503 of connecting a call illustrates a case where the first management terminal 15-1 has not received a call response from the user even after a lapse of a predetermined second time, like in the second embodiment. However, unlike the second embodiment, the third embodiment illustrates an example where the patient management server 13 attempts a call to the second management terminal 15-2 based on the priorities of the general contacts included in the list of the plurality of management terminals 15 corresponding to the patient management group.

Like in the first embodiment, the patient management server 13 continuously measures elapsed time, starting from the time it transmitted the calling signal to the first management terminal 15-1, and transmits first state information to the second management terminals 15-2 and 15-3 in Step 721, if a predetermined first time (e.g., 10 seconds) has elapsed, without receiving a call response signal from the user. Upon arrival at a predetermined second time, the patient management server 13 attempts a call to the second management terminals 15-2 and 15-3 based on the priories of the general contacts included in the list of the plurality of management terminals 15 corresponding to the patient management group, determining that the first management terminal 15-1 cannot respond to the call. Specifically, in Step 722, the patient management server 13 transmits second state information to the second management terminal 15-2 corresponding to the contact having the highest priority, depending on the priorities of the general contacts included in the list stored therein. The second state information includes information indicating the failure in connection to the first management terminal 15-1, information for requesting connection to the patient terminal 11, and information for requesting to start the second call to the patient terminal 11. In Step 723, upon receiving the second state information, the second management terminal 15-2 enables call connection to the patient terminal 11 that has been connected through the second call, and transmits a response signal ACK to the patient management server 13.

After transmitting the second state information, the patient management server 13 initializes the elapsed time and then re-measures the elapsed time. If no response signal ACK is received from the second management terminals 15-2 and 15-3 for the predetermined second time, the patient management server 13 repeatedly performs Steps 722 and 723 on the second management terminal 15-3 corresponding to the general contact having the next highest priority, which is included in the list.

Accordingly, the present invention provides an environment where managers may share information of interest to the patient.

In addition, managers may share information about the patient's condition, thereby increasing understanding between the patient and the managers, distributing patient management responsibilities concentrated on a specific manager, and assisting in the treatment and symptom management(e.g., autism) of the patient.

While the present invention has been described with reference to certain embodiments thereof, it will be understood by those skilled in the art that changes in form and details may be made therein without departing from the spirit and scope of the invention as defined by the appended claims and their equivalents.

## Claims

1. A group communication-based patient management method for operating patient management by a patient management server, comprising:
requesting connection of a first call between a patient terminal and a first management terminal corresponding to a representative contact in a patient management group list, and a second call between the patient terminal and a second management terminal corresponding to a general contact in the patient management group list;
connecting a call between the patient terminal and at least one of the first management terminal and second management terminal;
transmitting connection state information associated with the call connection, to one of the first management terminal and second management terminal, whose call to the patient terminal is not connected;
if termination of the call connection between the patient terminal and the at least one of the first management terminal and second management terminal, checking call result information; and
transmitting the call result information to the one of the first management terminal and second management terminal, whose call to the patient terminal is not connected.

2. The group communication-based patient management method of claim 1, wherein requesting connection comprises:
receiving, from the patient terminal, a request for connection of first call and second call to a management terminal corresponding to the patient management group list;
requesting connection of the first call between the patient terminal and the first management terminal; and
requesting connection of the second call between the patient terminal and the second management terminal.

3. The group communication-based patient management method of claim 2, wherein requesting connection further comprises:
receiving a request for the patient management group list from the patient terminal; and
providing the patient management group list to the patient terminal.

4. The group communication-based patient management method of claim 1, wherein requesting connection comprises:
receiving, from the patient terminal, a request for connection of first call and second call according to the patient management group list;
requesting connection of the first call to the patient terminal, from the first management terminal; and
requesting connection of the second call to the patient terminal, from the second management terminal.

5. The group communication-based patient management method of claim 4, wherein requesting connection of the first call comprises:
transmitting a calling signal for requesting a call connection to the first management terminal; and
transmitting a ringing signal to the patient terminal,
wherein requesting connection of the second call comprises transmitting a calling signal for requesting a call connection to the second management terminal, without transmitting a ringing signal to the patient terminal.

6. The group communication-based patient management method of claim 1, wherein connecting a call comprises:
if the first management terminal does not respond to the call connection request corresponding to a predetermined condition, terminating the first call between the patient terminal and the first management terminal; and
connecting the second call between the patient terminal and the second management terminal.

7. The group communication-based patient management method of claim 6, wherein connecting the second call between the patient terminal and the second management terminal comprises:
sending a message for requesting call connection to the second management terminal; and
if the second management terminal that sends a response to the sent message, sending a message for requesting to switch the second call to an enable state, so that a call session between the patient terminal and the second management terminal is connected.

8. The group communication-based patient management method of claim 1, wherein connecting a call comprises:
if the first management terminal responds to the call connection request corresponding to a predetermined condition, connecting a call session between the patient terminal and the first management terminal; and
terminating the second call between the patient terminal and the second management terminal.

9. The group communication-based patient management method of claim 6, wherein requesting call connection comprises:
if a first time corresponding to the predetermined condition expires, transmitting first state information including information about the first time to the second management terminal.

10. The group communication-based patient management method of claim 1, wherein connection state information includes at least one of an identifier of an unconnectable management terminal, an identifier of a connected management terminal, and an elapsed time for establishing connection.

11. The group communication-based patient management method of claim 1, wherein the call result information includes at least one of information about a patient's condition and information about a keyword of a conversation.

12. The group communication-based patient management method of claim 1, wherein checking call result information comprises:
requesting the call result information from one of the first management terminal and second management terminal, whose call is connected to the patient terminal; receiving the call result information from the management terminal whose call is connected to the patient terminal; and
checking the call result information.

13. A group communication-based patient management method for operating patient management by a management terminal using a patient terminal used by a patient, and the management terminal used by a user, which manages a patient and is included in a patient management group, comprising:
upon receiving a request for connection of a second call to the patient terminal, performing connection of a second call to the patient terminal;
receiving connection state information associated with connection of a first call between the patient terminal and a first management terminal;
providing the connection state information to the user; and
receiving call result information and providing the received call result information to the user.

14. The group communication-based patient management method of claim 13, further comprising:
receiving a request to enable connection of the second call; and establishing a call session to the patient terminal.

15. The group communication-based patient management method of claim 13, wherein the connection state information includes at least one of an identifier of an unconnectable management terminal, an identifier of a connected management terminal, and an elapsed time for establishing connection, and the call result information includes at least one of information about the patient's condition and information about a keyword of a conversation.
